# EUROPEAN PATENT SPECIFICATION

(11) **EP 4 431 947 B1**
(45) Date of publication and mention of the grant of the patent: **06.05.2026**
(21) Application number: 23397501.0
(22) Date of filing: 15.03.2023
(51) Int. Cl.: G01N 35/00, G01N 35/10, B01L 3/02

(54) **A METHOD OF QUANTIFYING AND PRESENTING AN EXPERIMENTAL ERROR**
VERFAHREN ZUR QUANTIFIZIERUNG UND DARSTELLUNG EINES EXPERIMENTELLEN FEHLERS
PROCÉDÉ DE QUANTIFICATION ET DE PRÉSENTATION D'UNE ERREUR EXPÉRIMENTALE

(43) Date of publication of application: 18.09.2024
(73) Proprietor: Sartorius Liquid Handling oy, 00880 Helsinki (FI)
(72) Inventor: JANTUNEN, Hannes, 00990 Helsinki (FI); VARHIMO, Emilia, 02200 Espoo (FI)
(74) Representative: Novagraaf International SA

(56) References cited:
- US-A1- 2009 000 403
- "Sample Preparation Techniques in Analytical Chemistry", vol. 162, 19 September 2003, JOHN WILEY & SONS, INC., Hoboken, NJ, USA, ISBN: 978-0-47-145781-7, article SOMENATH MITRA ET AL: "Sample Preparation: An Analytical Perspective", pages: 1 - 36, XP055110616, DOI: 10.1002/0471457817.ch1
- HANSON SONYA M ET AL: "Modeling error in experimental assays using the bootstrap principle: understanding discrepancies between assays using different dispensing technologies", JOURNAL OF COMPUTER-AIDED MOLECULAR DESIGN, SPRINGER NETHERLANDS, NL, vol. 29, no. 12, 17 December 2015 (2015-12-17), pages 1073 - 1086, XP035913018, ISSN: 0920-654X, [retrieved on 20151217], DOI: 10.1007/S10822-015-9888-6
- ANONYMOUS: "Andrew Alliance and Sartorius Collaborate to Provide Software-Connected Pipettes for Life Science Research", 8 February 2019 (2019-02-08), G�ttingen / Geneva, pages 1 - 3, XP093076001, Retrieved from the Internet <URL:https://www.sartorius.com/en/company/newsroom/corporate-news/60154-60154> [retrieved on 20230824]

## Description

### FIELD

The present application relates to quantifying errors in sample handling sequences, and particularly to quantifying and reducing errors accumulated in the course of multi-step sample handling sequences in a laboratory.

### BACKGROUND

Sample preparation and their analysis in a laboratory typically involve multi-step protocols during which multiple laboratory devices are needed. Such protocols may necessitate accurate determination of for example sample weight, sample volume and sample composition.

In liquid handling applications, typically several liquid handling devices for different liquid volumes and multiple dispensing steps are used in order to complete the desired application or workflow. Examples of such liquid handling applications include preparation of a dilution series and a multi-step kit. In addition to liquid handling steps also other handling and measuring methods, such as weighing, may be used in the same workflow.

Such multi-step and multi-device workflows are susceptible to accumulation of experimental error, which may seriously affect the accuracy of the ultimate result of the workflow. For example, if in a 5-step liquid dispensing sequence each dispensing step involves an experimental error of 10%, the total amount of liquid dispensed after the five steps will be in the range 59% to 161% of the targeted amount 100%.

It is known to reduce random and systematic experimental error in sample handling by calibrating the devices used and by striving to reduce any random variations in the sample handling procedure.

US 2009/0000403 A1 discloses a hybrid manual-electronic pipette with improved functionality, such as an LCD for displaying an error message.

### SUMMARY OF THE INVENTION

The invention is defined by the features of the independent claims. Some specific embodiments are defined in the dependent claims.

Various embodiments of the first aspect or the second aspect may comprise one or more features from the following bulleted list, wherein, in this list and throughout the rest of the description, those features that are required by the independent claims are not to be considered as optional:
- The method comprises, before or in said quantifying step, obtaining one or more of said experimental errors.
- Said obtaining comprises any of the following: estimating the experimental error, measuring the experimental error, or retrieving a pre-determined value for the experimental error, or any combination thereof.
- Said quantifying comprises quantifying by means of an empirically trained algorithm.
- Said quantifying comprises calculating a sum, for example a weighted sum, of individual experimental errors.
- Said quantifying comprises quantizing the individual experimental errors.
- The total experimental error takes into account at least two steps of a multi-step sample handling and/or analysis sequence comprising more than two steps.
- The sample handling and/or analysis sequence comprises or consists of a liquid handling and/or analysis sequence.
- Said sample handling and/or analysis sequence comprises one or more of the following steps: liquid handling, such as liquid aspiration, liquid dispensing and/or liquid pipetting, sample weighing, sample analysis, preferably at least liquid handling; and preferably a respective experimental error of said step is quantified.
- Said sample handling and/or analysis sequence comprises use of a laboratory device.
- Said laboratory device comprises a hand-held pipette, a liquid handling robot, a scale, an air pressure sensor, a temperature sensor, a humidity sensor, a spectrometer, or a sample analysis device.
- An experimental error in use of one or more of the following devices in the course of said sample handling and/or analysis sequence contributes to said total experimental error to be quantified and is taken into account: a hand-held pipette, a liquid handling robot, a scale, an air pressure sensor, a temperature sensor, a humidity sensor, a spectrometer, a sample analysis device.
- Experimental errors in use of at least two of the following devices in the course of said sample handling and/or analysis sequence contribute to said total experimental error to be quantified and are taken into account: a hand-held pipette, a liquid handling robot, a scale, an air pressure sensor, a temperature sensor, a humidity sensor, a spectrometer, and a sample analysis device.
- Said quantifying takes into account one or more of the following experimental errors during the sample handling and/or analysis sequence: a pipetting error, a weighing error, a sample analysis error, and an error in a concentration of a chemical or biological compound.
- Said output value comprises one or more of the following group: a parameter, an instruction and a recommendation, derived from any of said experimental errors and their combinations.
- The output device comprises a display of a laboratory device used in the sample handling and/or analysis sequence.
- The output device comprises a display of a hand-held laboratory device.
- The output device comprises a display of a mobile device, such as a display of a mobile phone or a tablet, which mobile device is used in the sample handling and/or analysis sequence.
- Said presenting is provided via a mobile application or a tablet software, a web-based service, or as a software integrated to laboratory device.
- Said output value comprises a recommendation to adjust a source of an individual experimental error, such as to adjust parameters related to said source, in order to reduce accumulation of the total experimental error.
- Said output value comprises a recommendation to use of a particular liquid handling device and/or a particular tip in a liquid handling device during execution of the current step or a forthcoming step of said sample handling and/or analysis sequence.
- Said presenting comprises presenting one or more charts, graphical symbols and/or alphanumeric symbols on the display.
- Said presenting comprises presenting a variable-size graphical symbol, such as an arrow or a circle, on the display.
- Said presenting comprises presenting a numerical uncertainty factor on the display.
- The method comprises: on the basis of the quantified experimental error, automatically locking a function of a laboratory device the use of which contributes to said error.
- The quantified experimental error or the output value is reported to a laboratory device the use of which contributes to said error.
- Said output value comprises a parameter, which is a control command or a correction factor for a laboratory device to reduce said total experimental error.
- Said reporting comprises reporting to a preceding step and/or to a forthcoming step.
- Said reporting comprises sending a control command or a correction factor to a laboratory device used in a preceding step or a forthcoming step.
- Said correction factor comprises a slope.
- The means for handling and/or analysing a sample comprises a liquid handling device.
- The output device is a display, audio means or audio-visual means.
- The device is a hand-held device, such as a hand-held electronic pipette.

Embodiments of the present invention aim at improving the known methods of reducing experimental error in sample handling processes.

At least some embodiments enable handling of experimental errors and their accumulation more effectively, particularly in the case of multi-step sample handling and analysis processes in which a plurality of laboratory devices and/or a plurality of users are involved.

An advantage of an embodiment is that a user may immediately react to accumulation of error and adjust or interrupt the ongoing sample handling process.

An advantage of an embodiment is that confirmed or predicted sources of error in sample handling may be indicated to a user during said sample handling.

An advantage of an embodiment is that a user is informed on error or error accumulation during or at sequential steps of sample handling. This enables the user to carry out particular actions or protocol amendments or even protocol interruption to reduce experimental error in a sample handling protocol and/or to avoid execution of protocols in which total experimental error would be unacceptably large.

An advantage of an embodiment is that a user may receive information of previous errors accumulated during earlier steps in a sample handling protocol and/or errors in the current and forthcoming steps.

An advantage of an embodiment is that unnecessary process steps may be avoided as the user may be informed of the uncertainty level in the sample handling and/or analysis sequence in the course of said sequence, and for example before beginning a particular step thereof.

An advantage of an embodiment is that accuracy and reproducibility particularly in multi-step and/or multi-device sample handling protocols may be improved.

### BRIEF DESCRIPTION OF THE DRAWINGS

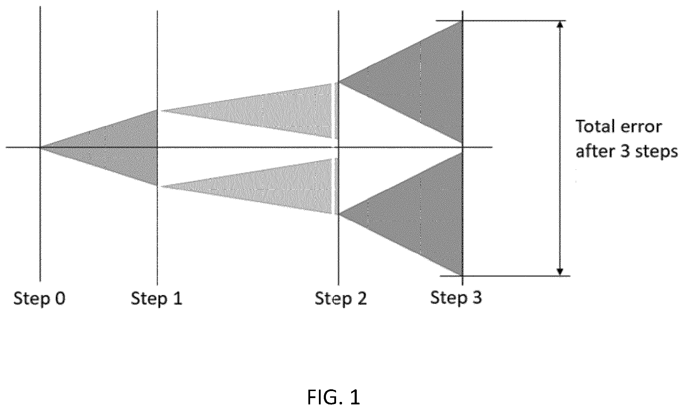

FIGURE 1 shows an exemplary visual presentation of accumulation of experimental error during a 3-step liquid dispensing sequence in accordance with an embodiment of the present invention.

### EMBODIMENTS

### Definitions

In the present context, the term "experimental error" refers to random and/or systematic experimental or observational error.

In the present context, the term "sample handling device" may refer to any device, typically a laboratory device, which is intended for handling a sample, for example a sample analysis device, a liquid handling device or a sample weighing device.

Typically, the term "laboratory device" refers to a sample handling device.

Typically, the term "sample handling" comprises sample handling and/or analysis.

In the present context, the term "display" may refer to any display which may be part of, or connected or integrated to a laboratory device, such as a sample handling device, in the course of a sample handling task or sequence.

It has been surprisingly observed that accuracy and/or reproducibility of sample handling in a laboratory may be significantly improved by the present method in which accumulation of experimental error during a sample handling protocol is quantified and presented to a user in the course of or even already in the beginning of execution of said protocol.

The experimental error to be quantified may comprise errors from various sources. The sources of experimental error may include instrumental, environmental, procedural, and human sources. The experimental error may comprise random and/or systematic error. Typically, experimental error comprises experimental error in sample handling and/or sample analysis.

In one embodiment, the present invention provides a method comprising: quantifying a total experimental error of a sample handling and/or analysis sequence, which sequence comprises at least two steps and respective individual experimental errors; presenting said total experimental error and/or presenting a parameter and/or an instruction and/or a recommendation derived from said total experimental error, to a user carrying out a step of said sample handling and/or analysis sequence.

### Sample handling sequence

The sample handing sequence typically comprises at least two sample handling steps or tasks.

A sample handling step or task may be a liquid pipetting task or a sample weighing task.

In one embodiment, the sample handling sequence comprises at least one liquid handling task or a liquid handling sequence.

The sample handling sequence may comprise a pipetting sequence involving multiple liquid aspirating and dispensing steps.

In one embodiment, the sample handling sequence comprises one or more of the following tasks: liquid handling, such as liquid aspiration, liquid dispensing or liquid pipetting; or sample handling, such as sample weighing or sample analysis, preferably at least liquid pipetting.

The sample handling sequence is performed using at least a hand-held pipette, and optionally a liquid handling robot, a scale, an air pressure sensor, a temperature sensor, a humidity sensor, a spectrometer, a sample analysis device.

### Quantifying error

Individual experimental errors in the use of these sample handling devices may be quantified. A total experimental error may be calculated from the individual experimental errors and presented to a user.

Experimental error may be quantified and presented as an absolute error or a relative error or both.

For example, the total error may take account of experimental error in a current sample handling and/or analysis step to be executed by the user and/or experimental error in previous and/or forthcoming sample handling and analysis steps of the same sample.

Taking account of previous sample handling steps when quantifying a total experimental error to be presented provides the advantage that the user may decide not to execute the next step at all if the already accumulated error is unacceptably large.

Taking account of current and forthcoming sample handling steps when quantifying a total experimental error to be presented provides the advantage that the user may adjust the execution to avoid accumulation of too large total experimental error. In this way it may be possible to complete the entire multi-step sample handling protocol with an acceptable experimental error.

Experimental errors in use of one or more devices in the course of a sample handling sequence, which devices may be called sample handling devices, may contribute to the total experimental error to be quantified. The one or more sample handling devices may comprise at least one of the following: a hand-held pipette, a liquid handling robot, a scale, an air pressure sensor, a temperature sensor, a humidity sensor, a spectrometer, or a sample analysis device.

The experimental error may relate to the accuracy of the sample handling device, to the action of sample handling and/or to the object of handling (the sample itself).

In some embodiments, the error quantification takes account of one or more experimental errors during a sample handling sequence. The one or more experimental errors may comprise at least one of the following: pipetting errors, weighing errors, sample analysis errors, and errors in concentrations of chemical or biological compounds.

In some embodiments the quantified error is reported or fed back to a sample handling or analysis device the use of which forms part of the sample handling task or sequence and thus contributes to accumulation of total experimental error.

For example, on the basis of the quantified error, it is possible to calculate a correction factor, such as a slope, and send it for use of a laboratory device used in a previous step or in a forthcoming step. A change of temperature between steps may be a source of systematic error which can be taken into account and corrected in this way.

The total error may be quantified by means of an empirically trained algorithm.

The algorithm may be a parameterized algorithm or a learning algorithm.

The total error may be quantified by calculating from individual errors related to individual steps in a multi-step sample handling sequence.

The quantifying may comprise for example: calculating a sum of individual errors, possibly after giving individual weights or probabilities to the errors to be added together, or possibly after quantizing the individual errors to be added together.

The means for quantifying the error may comprise a computing device, typically integrated to a sample handling device, such as a hand-held sample handling device. Said computing device is preferably configured to quantify an accumulated error from individual experimental errors in steps or stages of a sample handling sequence, which sample handling sequence may include a liquid handling task carried out by a liquid handling device.

In some embodiments, the computing device has been integrated into a liquid handling device, such as a hand-held liquid handling device or an automated liquid handling station, preferably into a hand-held liquid handling device.

By means of quantifying and presenting the total experimental error, the embodiments provide information on current status of the sample handling and/or possible estimations for the further steps. This enables the user to follow-up and understand the effect of accumulation of experimental error and in this way to improve reliability of results.

### Output value

The method comprises presenting the quantified error(s). The method comprises presenting an output value or values derived from the error(s).

The type of output values may be tailored for example on the basis of the user and/or on the basis of error accumulation speed.

Educated users may benefit from directly receiving error information, whereas non-educated users may prefer receiving output values comprising concrete or practical instructions.

As an example, if the total error is accumulating slowly, the output values may comprise more general instructions than in a case where a sudden increase of total error is being observed.

The method preferably comprises showing an output value by an output device, such as a display, based on assessment of the individual experimental errors and the total experimental error.

The method preferably comprises showing an instruction and/or a recommendation to the user, for example by an output device, such as a display. The display may be part of, or connected or integrated to a laboratory device, such as a sample handling device, in the course of a sample handling task or sequence. Said instruction and/or said recommendation preferably guides the user to reduce said quantified error in execution of the sample handling task in the current step or the forthcoming steps of the sample handling and/or analysis sequence.

Said recommendation may relate to a step or device in which adjustments have the largest potential to reduce the total error.

Said recommendation may relate to a step or device in which adjustments are not susceptible of increasing errors in other steps or other devices.

Said recommendation may comprise recommending use of a particular liquid handling device in a liquid handling task and/or a particular tip in a liquid handling device during execution of the current step or a forthcoming step of said sample handling and/or analysis sequence.

For example, the selection of a tip volume may be a source of systematic error, particularly with tips for automated liquid handling robots.

Further, if the same tip is being used for a long time, a systematic error may begin to accumulate.

In air displacement pipettes, it may be possible to detect errors that are typical or specific for their use, for example if the user has not pre-wetted the tip or does not work at temperature equilibrium. Such errors may be indicated to the user. It may also be possible to take account of these errors by applying a correction factor.

Said recommendation may comprises recommending use of a particular liquid handling parameter, such as liquid volume to be dispensed, in a liquid handling task.

Preferably said recommendation guides a user to actions that are adapted to reduce experimental error in the current and/or any of the forthcoming steps of a sample handling procedure. Said recommendation may guide a user to actions that are adapted to reduce total experimental error of an entire sample handling procedure. Said action may comprise continuing the current procedure with adjusted parameters or adjusted equipment. Said action may comprise interrupting and relaunching of the current sample handling step or the entire sample handling and/or analysis sequence.

In some embodiments, such interrupting or relaunching may be avoided by reporting, as an output value, a parameter, such as a correction factor, to a forthcoming step.

In an embodiment, an output value is based on the experimental error of the current step.

In an embodiment, an output value is based on accumulated error, i.e. the experimental error of the current step and the experimental error of at least one previous step, preferably all previous steps.

In an embodiment, an output value is based on accumulated error and estimated forthcoming error, i.e. the experimental error of the current step, all previous steps and all forthcoming steps.

In an embodiment, if a threshold value for the error is approached or exceeded, an output value is generated.

The threshold value may be a predetermined threshold value or it may be a learning threshold, such as a threshold determined by a learning algorithm.

The threshold value may be a threshold value for an error of an individual step or a threshold value for an error accumulated during several steps.

In one embodiment, the recommendation comprises recommending use of a particular combination of laboratory devices, such as liquid handling devices, to minimize the total experimental error.

Said combination may comprise a combination of at least two sample handling devices, which may be the same or different.

Said combination may comprise a combination of at least three sample handling devices, such as at least four sample handling devices.

Said combination may comprise at least an automated liquid handling station or a hand-held pipette. For example, said combination may comprise a hand-held pipette and an automated liquid handling station. As another example, said combination may comprise an automated liquid handling station and a scale. As another example, said combination may comprise a hand-held pipette and an automated liquid handling station.

In an embodiment, the recommendation comprises recommending use of an electronic pipette, an automated liquid handling station, a mechanically actuated pipette or any combination thereof.

In an embodiment, the recommendation comprises recommending optimal dispensing volumes in the preparation of a dilution series: for example, whether to prepare a 1:30 dilution by first diluting 1:3 and thereafter 1:3, or alternatively by diluting twice about 1 : 5.47.

In an embodiment, the recommendation comprises recommending use of a tip by a particular tip manufacturer.

In an embodiment, the recommendation comprises recommending change of tip.

In an embodiment, the recommendation comprises recommending change of liquid handling equipment or sample weighing equipment.

In an embodiment, the recommendation comprises recommending interrupting and re-execution of the sample handling sequence, for example by using a different liquid handling device or a different scale or different pipetting parameters.

### Presenting error

The error is presented by an output device, such as an electronic output device. Typically, the output device is a display.

In an embodiment, the presenting is carried out by an output device of a laboratory device which is operable by the user, and/or which is used for the sample handling and/or sequence analysis.

Preferably, the error is presented in a hand-held device. The hand-held device may be a hand-held laboratory device that the user may operate to execute the sample handling sequence.

Most preferably, the error is presented on a display of a hand-held laboratory device.

In an embodiment, the presenting is carried out by a display integrated to a liquid handling device, such as an electronic pipette or an automated liquid handling station.

In an embodiment, the presenting is carried out by a display of a sample handling device used in the sample handling sequence.

In some embodiments, the presenting may be carried out by any suitable means or an output device, such as by visual means, by audio-visual means, or by audio means. Use of visual means enables that more information, particularly alphanumerical information, can be presented to the user about the magnitude and nature of an error. The advantage of audio means is that the user need not observe any display.

In an embodiment, the presenting comprises vibrating of the hand-held laboratory device.

Preferably, the presenting is carried out via a device held by or worn by or located in the sight of a user who is executing a sample handling and/or analysis task to which the presented experimental error relates. The device for presenting may be connected or integrated to the sample handling device, or to a control device of such.

In an embodiment, the presenting is carried out by an output device of a mobile device that is available to the user during execution of the sample handling sequence. The mobile device may be a mobile phone, a laptop or a tablet. The mobile device may be able to receive information to be presented (output) via a wireless or wired connection.

Said presenting may be provided via wireless or wired connection via a mobile application or a tablet software or a web-based service, or via a software integrated to a sample handling device.

Said presenting may comprise showing one or more charts, graphical symbols and/or alphanumeric symbols on a display of a sample handling device.

Said presenting may comprise presenting a variable-size graphical symbol, such as an arrow or a circle, on a display of sample handling device.

Preferably said presenting comprises presenting a numerical uncertainty factor to a user for example on a display of a sample handling device, which may be a hand-held device.

In an embodiment, said uncertainty factor may be derived from a total experimental error of an entire sample handling and/or analysis sequence.

In an embodiment, said uncertainty factor may be derived from an experimental error of an individual sample handling step.

In an embodiment, said uncertainty factor may be derived from accumulated experimental error.

In an embodiment, said uncertainty factor may be derived from estimated experimental error of the current step and/or the forthcoming steps.

The method may comprise automated locking of one or more functions of a sample handling device, for example temporarily. The advantage is that execution of the protocol and accumulation of error is automatically stopped without having to rely on the user's reaction to the presented information.

The present invention also provides a system configured to perform the method.

The system comprises at least a hand-held pipette and optionally one or more of the following devices:
a liquid handling robot, a scale, an air pressure sensor, a temperature sensor, a humidity sensor, a spectrometer, a sample analysis device, preferably at least a hand-held device, such as a pipette. In addition the system may comprise a device for controlling or operating the system, or liquid handling execution, which may include sample handling and/or sequence analysis. The device for controlling may be called a controller. The controller may be a computer or a mobile device.

Said devices may be connected to each other by means of a wireless connection, such as a Bluetooth connection. In this way it is possible to transmit data, such as error data, such as the quantified total error, between the devices, for example in cases where the total error is quantified by a device that is not the same as the device that will be presenting the error to the user.

In an embodiment, the total experimental error is calculated by a hand-held liquid handling device, and said error is presented by the hand-held liquid handling device.

In an embodiment, the total experimental error is calculated by an automated liquid handling station, and said error is presented by a hand-held liquid handling device.

In an embodiment, the total experimental error is calculated by an automated sample analysis device or station, and said error is presented by a hand-held liquid handling device.

In some embodiments, data retrieved from one or more laboratory devices may be shown on the display of a hand-held pipette. Such data may comprise experimental errors and/or output values derived therefrom. The display of the pipette may thus present a combination of errors and/or output values. In this way it is possible to illustrate to the user the accumulation and sources of errors in previous steps in detail and in good time, for example in a situation where the total error slowly accumulates and approaches the set thresholds.

The present invention may also provide a sample handling device comprising means for performing the method. In an embodiment the sample handling device comprises: a computing device configured to quantify said total experimental error, and an output device, such as a display, configured to present said total experimental error.

FIGURE 1 shows an exemplary visual presentation of accumulation of experimental error during a 3-step liquid dispensing sequence in accordance with an embodiment of the present invention. Individual experimental errors in each of the three steps (Step 1, Step 2 and Step 3) and the total experimental error after the three steps are presented graphically.

If the Steps 1, 2 and 3 are different, such as performed by different types of devices or involving different type of sample handling actions, it may be preferable to carry out quantization of the errors before adding them together.

### Examples

In one example, the sample handling and/or analysis sequence comprises a programmed pipetting sequence. The pipette is configured to follow accumulation of experimental error and to estimate the total experimental error of the entire programmed sequence. Thus the pipette quantifies or determines experimental errors of past steps, calculates the accumulated error, optionally compares to respective threshold values, estimates forthcoming errors, and calculates (estimates) the total error of the entire sequence. The estimate may change or be updated as the sequence progresses. The pipette presents the accumulated experimental error and/or the estimated total experimental error to the user. The user is able to assess whether it is possible to complete the programmed sequence with an acceptable level of uncertainty. Alternatively, the pipette may automatically generate such assessment and present it to the user.

In one example, one or more of the following are taken into account when quantifying the total experimental error in a liquid handling sequence: tip, liquid to be dispensed, number of doses to be dispensed, user, air humidity, ambient temperature. A threshold value or an acceptable value may be associated with each individual source of error.

Handling of solvents in the same space in which other sample handling and/or analysis procedures are carried out increases experimental error in said procedures. In one example, a volatile organic compound (VOC) sensor is used to detect concentrations of volatile organic compounds in the air of a laboratory. The detected concentrations are taken into account when quantifying the total experimental error in said sample handling and/or analysis procedure.

In one example, the sample handling and/or analysis sequence includes a liquid handling task, a polymerase chain reaction (PCR) analysis task and a protein separation task. Accumulation of experimental error is presented to the user during the execution of said steps, for example as an uncertainty factor. In this way the user is able to assess on the basis of the magnitude of the uncertainty factor whether it is grounded to continue the execution of said tasks and for example to proceed from one task to the next task. In addition or alternatively, a recommendation is presented. The recommendation may comprise an indication based on the determined uncertainty factor. The indication may refer to an error source, and optionally to an adjustment to be made in order to bring the uncertainty factor of the error source to an acceptable level. The acceptable level refers to an error level which is not considered detrimental for continuation of the execution of the tasks.

It is to be understood that the embodiments of the invention disclosed are not limited to the particular structures, process steps, or materials disclosed herein, but are extended to equivalents thereof as would be recognized by those ordinarily skilled in the relevant arts. It should also be understood that terminology employed herein is used for the purpose of describing particular embodiments only and is not intended to be limiting.

Reference throughout this specification to "one embodiment" or "an embodiment" means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment.

As used herein, a plurality of items, structural elements, compositional elements, and/or materials may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary. In addition, various embodiments and example of the present invention may be referred to herein along with alternatives for the various components thereof. It is understood that such embodiments, examples, and alternatives are not to be construed as de facto equivalents of one another, but are to be considered as separate and autonomous representations of the present invention.

Furthermore, the described features, structures, or characteristics may be combined in any suitable manner in one or more embodiments. In the following description, numerous specific details are provided, such as examples of lengths, widths, shapes, etc., to provide a thorough understanding of embodiments of the invention. One skilled in the relevant art will recognize, however, that the invention can be practiced without one or more of the specific details, or with other methods, components, materials, etc. In other instances, well-known structures, materials, or operations are not shown or described in detail to avoid obscuring aspects of the invention.

While the forgoing examples are illustrative of the principles of the present invention in one or more particular applications, it will be apparent to those of ordinary skill in the art that numerous modifications in form, usage and details of implementation can be made without the exercise of inventive faculty, and without departing from the principles and concepts of the invention. Accordingly, it is not intended that the invention be limited, except as by the claims set forth below.

The verbs "to comprise" and "to include" are used in this document as open limitations that neither exclude nor require the existence of also un-recited features. The features recited in depending claims are mutually freely combinable unless otherwise explicitly stated. Furthermore, it is to be understood that the use of "a" or "an", i.e. a singular form, throughout this document does not exclude a plurality.

### INDUSTRIAL APPLICABILITY

The present invention is industrially applicable at least in sample handling and/or analysis methods, such as preparation, pipetting, and/or analysis of samples, in a laboratory environment.

## Claims

1. A method for sample handling and/or analysis sequence comprising:
- quantifying a total experimental error of a multi-step sample handling and/or analysis sequence comprising at least two steps, including quantifying respective individual experimental errors of said at least two steps; and
- presenting an output value derived from said total experimental error by an output device, the output device comprising a display of a hand-held pipette, and the output value comprising a recommendation for guiding the user to reduce said quantified error in execution of the sample handling task in the current step or the forthcoming steps of the sample handling and/or analysis sequence; and
reporting the output value to a laboratory device the use of which contributes to said error.

2. The method according to claim 1 wherein said quantifying comprises any of the following: estimating the experimental error, measuring the experimental error, or retrieving a pre-determined value for the experimental error, or any combination thereof.

3. The method according to any of the preceding claims, wherein said quantifying comprises quantifying by means of an empirically trained algorithm, or calculating a sum, for example a weighted sum, of individual experimental errors, or quantizing the individual experimental errors.

4. The method according to any of the preceding claims, wherein the sample handling and/or analysis sequence comprises or consists of a liquid handling and/or analysis sequence.

5. The method according to any of the preceding claims, wherein an experimental error in use of one or more of the following devices in the course of said sample handling and/or analysis sequence contributes to said total experimental error to be quantified and is taken into account: a hand-held pipette, a liquid handling robot, a scale, an air pressure sensor, a temperature sensor, a humidity sensor, a spectrometer, a sample analysis device.

6. The method according to any of the preceding claims, wherein said output value comprises one or more of the following group: a parameter and an instruction, derived from any of said experimental errors and their combinations.

7. The method according to any of the preceding claims, wherein the recommendation for guiding a user to actions that are adapted to reduce:
- experimental error in the current and/or any of the forthcoming steps of a sample handling procedure; and/or
- total experimental error of an entire sample handling procedure.

8. The method according to claim 7, wherein said actions comprise continuing the current procedure with adjusted parameters or adjusted equipment..

9. The method according to any of the preceding claims, wherein
said output value comprises a recommendation to adjust a source of an individual experimental error, such as to adjust parameters related to said source, in order to reduce accumulation of the total experimental error, or
said output value comprises a recommendation to use a particular liquid handling device and/or a particular tip in a liquid handling device during execution of the current step or a forthcoming step of said sample handling and/or analysis sequence.

10. The method according to any of the preceding claims, wherein said presenting comprises presenting one or more of the following by the output device: a chart, a graphical symbol, an alphanumeric symbol or any combination thereof.

11. The method according to any of the preceding claims, wherein said presenting comprises presenting a numerical uncertainty factor by the output device.

12. The method according to any of the preceding claims, comprising:
on the basis of the quantified experimental error, automatically locking or adjusting a function of a laboratory device the use of which device contributes to said error.

13. The method according to any of the preceding claims, wherein
the quantified experimental error or the output value is reported to a laboratory device the use of which contributes to said error; and
said output value comprises a parameter, which is a control command or a correction factor for the laboratory device to reduce said experimental error.

14. A device comprising means for performing the method according to any of claims 1 to 13, comprising:
- means for handling and/or analysing a sample comprising one or more sample handling devices;
- a computing device configured to quantify said experimental errors, and
- an output device comprising a display of a hand-held pipette configured to present said total experimental error and/or said output value.

15. The device according to claim 14, wherein
the means for handling and/or analysing a sample comprises a liquid handling device, and the output device comprises audio means or audio-visual means.

## Patentansprüche

1. Verfahren zur Probenhandhabung und/oder -analyse, umfassend:
- das Quantifizieren eines gesamten experimentellen Fehlers einer mehrstufigen Probenhandhabungs- und/oder Analysesequenz, die mindestens zwei Stufen umfasst, einschließlich des Quantifizierens jeweiliger individueller experimenteller Fehler der mindestens zwei Stufen; und
- das Darstellen eines Ausgabewerts, der von dem gesamten experimentellen Fehler abgeleitet ist, mittels eines Ausgabegeräts, wobei das Ausgabegerät eine Anzeige einer Handpipette umfasst und der Ausgabewert eine Empfehlung umfasst, um den Benutzer anzuleiten, den quantifizierten Fehler bei der Ausführung der Probenhandhabungsaufgabe in der aktuellen Stufe oder den bevorstehenden Stufen der Probenhandhabungs- und/oder Analysesequenz zu verringern; und das Melden des Ausgabewerts an ein Laborgerät, dessen Verwendung zu dem Fehler beiträgt.

2. Verfahren nach Anspruch 1, wobei das Quantifizieren eines der folgenden Elemente umfasst: das Schätzen des experimentellen Fehlers, das Messen des experimentellen Fehlers oder das Abrufen eines vorbestimmten Werts für den experimentellen Fehler, oder eine beliebige Kombination davon.

3. Verfahren nach einem der vorhergehenden Ansprüche, wobei das Quantifizieren das Quantifizieren mittels eines empirisch trainierten Algorithmus oder das Berechnen einer Summe, beispielsweise einer gewichteten Summe, individueller experimenteller Fehler oder das Quantisieren der individuellen experimentellen Fehler umfasst.

4. Verfahren nach einem der vorhergehenden Ansprüche, wobei die Probenhandhabungs- und/oder Analysesequenz eine Flüssigkeitshandhabungs- und/oder Analysesequenz umfasst oder daraus besteht.

5. Verfahren nach einem der vorhergehenden Ansprüche, wobei ein experimenteller Fehler bei der Verwendung eines oder mehrerer der folgenden Geräte im Verlauf der Probenhandhabungs- und/oder Analysesequenz zu dem zu quantifizierenden gesamten experimentellen Fehler beiträgt und berücksichtigt wird: eine Handpipette, ein Flüssigkeitshandhabungsroboter, eine Waage, ein Luftdrucksensor, ein Temperatursensor, ein Feuchtigkeitssensor, ein Spektrometer, ein Probenanalysegerät.

6. Verfahren nach einem der vorhergehenden Ansprüche, wobei der Ausgabewert eines oder mehrere Elemente der folgenden Gruppe umfasst: einen Parameter und eine Anweisung, die von einem der experimentellen Fehler und deren Kombinationen abgeleitet sind.

7. Verfahren nach einem der vorhergehenden Ansprüche, wobei die Empfehlung zur Anleitung eines Benutzers darauf ausgerichtet ist, Folgendes zu verringern:
- den experimentellen Fehler in der aktuellen und/oder einer der bevorstehenden Stufen eines Probenhandhabungsverfahrens; und/oder
- den gesamten experimentellen Fehler eines vollständigen Probenhandhabungsverfahrens.

8. Verfahren nach Anspruch 7, wobei die Maßnahmen die Fortsetzung des aktuellen Verfahrens mit angepassten Parametern oder angepasster Ausrüstung umfassen..

9. Verfahren nach einem der vorhergehenden Ansprüche, wobei
der Ausgabewert eine Empfehlung umfasst, eine Quelle eines individuellen experimentellen Fehlers anzupassen, wie etwa die Anpassung von Parametern, die sich auf die Quelle beziehen, um die Anhäufung des gesamten experimentellen Fehlers zu verringern, oder
der Ausgabewert eine Empfehlung umfasst, ein bestimmtes Flüssigkeitshandhabungsgerät und/oder eine bestimmte Spitze in einem Flüssigkeitshandhabungsgerät während der Ausführung der aktuellen Stufe oder einer bevorstehenden Stufe der Probenhandhabungs- und/oder Analysesequenz zu verwenden.

10. Verfahren nach einem der vorhergehenden Ansprüche, wobei das Darstellen das Darstellen eines oder mehrerer der folgenden Elemente durch das Ausgabegerät umfasst: ein Diagramm, ein grafisches Symbol, ein alphanumerisches Symbol oder eine beliebige Kombination davon.

11. Verfahren nach einem der vorhergehenden Ansprüche, wobei das Darstellen das Darstellen eines numerischen Unsicherheitsfaktors durch das Ausgabegerät umfasst.

12. Verfahren nach einem der vorhergehenden Ansprüche, umfassend:
auf der Grundlage des quantifizierten experimentellen Fehlers, das automatische Sperren oder Anpassen einer Funktion eines Laborgeräts, dessen Verwendung zu dem Fehler beiträgt.

13. Verfahren nach einem der vorhergehenden Ansprüche, wobei
der quantifizierte experimentelle Fehler oder der Ausgabewert an ein Laborgerät gemeldet wird, dessen Verwendung zu dem Fehler beiträgt; und
der Ausgabewert einen Parameter umfasst, der ein Steuerbefehl oder ein Korrekturfaktor für das Laborgerät ist, um den experimentellen Fehler zu verringern.

14. Vorrichtung, umfassend Mittel zur Durchführung des Verfahrens nach einem der Ansprüche 1 bis 13, umfassend:
- Mittel zum Handhaben und/oder Analysieren einer Probe, umfassend ein oder mehrere Probenhandhabungsgeräte;
- ein Rechengerät, das zum Quantifizieren der experimentellen Fehler konfiguriert ist, und
- ein Ausgabegerät, umfassend eine Anzeige einer Handpipette, die konfiguriert ist, um den gesamten experimentellen Fehler und/oder den Ausgabewert darzustellen.

15. Vorrichtung nach Anspruch 14, wobei
die Mittel zum Handhaben und/oder Analysieren einer Probe ein Flüssigkeitshandhabungsgerät umfassen, und
das Ausgabegerät Audiomittel oder audiovisuelle Mittel umfasst.

## Revendications

1. Procédé de manipulation et/ou d'analyse d'échantillons comprenant :
- la quantification d'une erreur expérimentale totale d'une séquence de manipulation et/ou d'analyse d'échantillons en plusieurs étapes comprenant au moins deux étapes, incluant la quantification d'erreurs expérimentales individuelles respectives desdites au moins deux étapes ; et
- la présentation d'une valeur de sortie dérivée de ladite erreur expérimentale totale par un dispositif de sortie, le dispositif de sortie comprenant un affichage d'une pipette portative, et la valeur de sortie comprenant une recommandation pour guider l'utilisateur afin de réduire ladite erreur quantifiée lors de l'exécution de la tâche de manipulation d'échantillons dans l'étape actuelle ou les étapes à venir de la séquence de manipulation et/ou d'analyse d'échantillons ; et le signalement de la valeur de sortie à un dispositif de laboratoire dont l'utilisation contribue à ladite erreur.

2. Procédé selon la revendication 1, dans lequel ladite quantification comprend l'un quelconque des éléments suivants : l'estimation de l'erreur expérimentale, la mesure de l'erreur expérimentale, ou la récupération d'une valeur prédéterminée pour l'erreur expérimentale, ou toute combinaison de ceux-ci.

3. Procédé selon l'une quelconque des revendications précédentes, dans lequel ladite quantification comprend la quantification au moyen d'un algorithme entraîné empiriquement, ou le calcul d'une somme, par exemple une somme pondérée, d'erreurs expérimentales individuelles, ou la quantisation des erreurs expérimentales individuelles.

4. Procédé selon l'une quelconque des revendications précédentes, dans lequel la séquence de manipulation et/ou d'analyse d'échantillons comprend ou consiste en une séquence de manipulation et/ou d'analyse de liquides.

5. Procédé selon l'une quelconque des revendications précédentes, dans lequel une erreur expérimentale liée à l'utilisation d'un ou plusieurs des dispositifs suivants au cours de ladite séquence de manipulation et/ou d'analyse d'échantillons contribue à ladite erreur expérimentale totale à quantifier et est prise en compte : une pipette portative, un robot de manipulation de liquides, une balance, un capteur de pression d'air, un capteur de température, un capteur d'humidité, un spectromètre, un dispositif d'analyse d'échantillons.

6. Procédé selon l'une quelconque des revendications précédentes, dans lequel ladite valeur de sortie comprend un ou plusieurs éléments du groupe suivant : un paramètre et une instruction, dérivés de l'une quelconque desdites erreurs expérimentales et de leurs combinaisons.

7. Procédé selon l'une quelconque des revendications précédentes, dans lequel la recommandation visant à guider un utilisateur est adaptée pour réduire :
- l'erreur expérimentale dans l'étape actuelle et/ou l'une quelconque des étapes à venir d'une procédure de manipulation d'échantillons ; et/ou
- l'erreur expérimentale totale d'une procédure de manipulation d'échantillons entière.

8. Procédé selon la revendication 7, dans lequel lesdites actions comprennent la poursuite de la procédure actuelle avec des paramètres ajustés ou un équipement ajusté..

9. Procédé selon l'une quelconque des revendications précédentes, dans lequel
ladite valeur de sortie comprend une recommandation d'ajuster une source d'une erreur expérimentale individuelle, telle que d'ajuster des paramètres liés à ladite source, afin de réduire l'accumulation de l'erreur expérimentale totale, ou
ladite valeur de sortie comprend une recommandation d'utiliser un dispositif de manipulation de liquides particulier et/ou un embout particulier dans un dispositif de manipulation de liquides pendant l'exécution de l'étape actuelle ou d'une étape à venir de ladite séquence de manipulation et/ou d'analyse d'échantillons.

10. Procédé selon l'une quelconque des revendications précédentes, dans lequel ladite présentation comprend la présentation d'un ou plusieurs des éléments suivants par le dispositif de sortie : un diagramme, un symbole graphique, un symbole alphanumérique ou toute combinaison de ceux-ci.

11. Procédé selon l'une quelconque des revendications précédentes, dans lequel ladite présentation comprend la présentation d'un facteur d'incertitude numérique par le dispositif de sortie.

12. Procédé selon l'une quelconque des revendications précédentes, comprenant :
sur la base de l'erreur expérimentale quantifiée, le verrouillage ou l'ajustement automatique d'une fonction d'un dispositif de laboratoire dont l'utilisation dudit dispositif contribue à ladite erreur.

13. Procédé selon l'une quelconque des revendications précédentes, dans lequel
l'erreur expérimentale quantifiée ou la valeur de sortie est signalée à un dispositif de laboratoire dont l'utilisation contribue à ladite erreur ; et
ladite valeur de sortie comprend un paramètre, qui est une commande de contrôle ou un facteur de correction pour le dispositif de laboratoire afin de réduire ladite erreur expérimentale.

14. Dispositif comprenant des moyens pour mettre en œuvre le procédé selon l'une quelconque des revendications 1 à 13, comprenant :
- des moyens de manipulation et/ou d'analyse d'un échantillon comprenant un ou plusieurs dispositifs de manipulation d'échantillons ;
- un dispositif informatique configuré pour quantifier lesdites erreurs expérimentales, et
- un dispositif de sortie comprenant un affichage d'une pipette portative configuré pour présenter ladite erreur expérimentale totale et/ou ladite valeur de sortie.

15. Dispositif selon la revendication 14, dans lequel
les moyens de manipulation et/ou d'analyse d'un échantillon comprennent un dispositif de manipulation de liquides, et
le dispositif de sortie comprend des moyens audio ou des moyens audiovisuels.
